# EUROPEAN PATENT SPECIFICATION

(11) **EP 1 538 989 B1**
(45) Date of publication and mention of the grant of the patent: **17.03.2010**
(21) Application number: 03766304.4
(22) Date of filing: 25.07.2003
(51) Int. Cl.: A61B 17/00

(54) **APPARATUS FOR SEALING PUNCTURES IN BLOOD VESSELS**
GERÄT ZUR ABDICHTUNG VON PUNKTIONSSTELLEN IN BLUTGEFÄSSEN
APPAREIL PERMETTANT DE FERMER DES PERFORATIONS DANS DES VAISSEAUX SANGUINS

(30) Priority: 01.08.2002 US 401222 P
(43) Date of publication of application: 15.06.2005
(73) Proprietor: Abbott Laboratories Vascular Enterprises Limited, Terrace, Dublin 2 (IE)
(72) Inventor: VON OEPEN, Randolf, Los Altos Hills, CA 94024 (US); SEIBOLD, Gerd, 72119 Ammerbuch (DE); JOERGENSEN, Ib Erling, 72401 Haigerloch (DE); MICHLITSCH, Kenneth, J., Livermore, CA 94550 (US); NIELSEN, Stevan, 72108 Rottenburg (DE); CONZELMANN, Tommy, Dublin 2 (IE); QUINT, Bodo, 72108 Rottenburg (DE)
(74) Representative: Peters, Hajo
(86) International application number: PCT/EP2003/008245
(87) International publication number: WO 2004/012601

(56) References cited:
- EP-A- 0 367 516
- US-A- 3 411 505
- US-A- 5 411 520
- US-A- 5 810 884
- US-B1- 6 371 974

## Description

### Field Of The Invention

The present invention relates to apparatus for sealing punctures in blood vessels. More specifically, the invention relates to one or more devices that may be deployed in tissue distal of a vessel having a puncture to apply an internal compressive force upon the vessel to facilitate sealing of the puncture.

### Background of the Invention

A large number of medical diagnostic and therapeutic procedures involve the percutaneous introduction of instrumentation into the blood vessel. For example, coronary angioplasty, angiography, atherectomy, stenting, and numerous other procedures often involve accessing the vasculature through placement of a catheter or other device in a patient's femoral artery or other blood vessel. Once the procedure is completed and the catheter or other diagnostic or therapeutic device is removed, bleeding from the resultant vascular puncture must be stopped.

Traditionally, a medical practitioner applies external pressure to the puncture site to stem bleeding until hemostasis occurs (i.e., when the clotting and tissue rebuilding have sealed the puncture). This method, however, presents numerous problems. In some instances, this pressure must be applied for up to an hour or more, during which time the patient is uncomfortably immobilized. In addition, there exists a risk of hematoma since bleeding from the puncture may continue until sufficient clotting occurs, particularly if the patient moves during the clotting process. Furthermore, application of external pressure to stop bleeding may be unsuitable for patients with substantial amounts of subcutaneous adipose tissue since the skin surface may be a considerable distance from the puncture site, thereby rendering external compression less effective.

Another traditional approach to subcutaneous puncture closure comprises having a medical practitioner internally suture the vessel puncture. This method, however, often requires a complex procedure and requires considerable skill by the medical practitioner.

Apparatus and methods also are known in which a plug is introduced into the vessel puncture to cover the puncture and promote hemostasis. One example of such a plug is described in U.S. Patent No. 5,061,274 to Kensey and comprises a plug made from animal-derived collagen. Such apparatus may be unsuitable for some patients due to an adverse immunological reaction to animal-derived collagen. Furthermore, a plug inserted into the puncture tract may be dislodged into the vessel during the healing process due to the application of pressure to the wound, potentially causing stenosis of the vessel.

U.S. Patent Nos. 5,411,520 and 5,810,884 disclose further examples of an apparatus and method in which a plug member is introduced into the puncture site to promote hemostasis. In U.S. 5,411,520 an anchor member is designed to be secured inside the blood vessel. In U.S. 5,810,884 a cable attached to a buttressing support member outside of the blood vessel is used to secure the plug member in position.

In view of the drawbacks of previously known devices, it would be desirable to provide apparatus for sealing a puncture within a vessel that provides an internal compressive force upon the vessel.

It also would be desirable to provide apparatus for sealing a puncture within a vessel that may provide the internal compressive force in conjunction with an external compressive force applied to an exterior surface of a patient's skin.

It further would be desirable to provide apparatus for sealing a puncture within a vessel that does not require a physician to manually maintain the compressive forces imposed.

It still further would be desirable to provide apparatus for sealing a puncture within a vessel that is biodegradable.

### Summary of the Invention

In view of the foregoing, it is an object of the present invention to provide apparatus for sealing a puncture within a vessel that provides an internal compressive force upon the vessel.

It also is an object of the present invention to provide apparatus for sealing a puncture within a vessel that may provide the internal compressive force in conjunction with an external compressive force applied to an exterior surface of a patient's skin.

It further is an object of the present invention to provide apparatus for sealing a puncture within a vessel that does not require a physician to manually maintain the compressive forces imposed.

It further is an object of the present invention to provide apparatus for sealing a puncture within a vessel that is biodegradable.

The present invention provides an apparatus as defined in claim 1 or claim 15. Preferred features of the invention are recited in the dependent claims. Thus, the invention provides an apparatus for sealing a puncture within a vessel that comprises at least one device that may be deployed in tissue distal of the vessel to apply an internal compressive force upon the vessel. In the context of the present invention, the term "internal compressive force" refers generally to a force imposed at a location distal of a vessel and occurring in proximal direction toward a skin puncture. By contrast, an "external compressive force" is defined herein as a force imposed at a location near a skin puncture and occurring in a distal direction toward the vessel.

In a first embodiment of the present invention, the device preferably comprises a bar having a bore and a filament disposed through the bore. The bar and the filament are provided in a contracted state within a delivery sheath, for example, a hypodermic needle, having proximal and distal ends and a sharpened tip at the distal end. The tip of the delivery sheath is advanced distally to pass through the original puncture, which had previously been formed in a proximal lateral surface of the vessel. The tip further is advanced to pierce through a distal lateral surface of the vessel and is positioned distal of the vessel.

The bar then is ejected from within the confines of the delivery sheath, e. g. by advancing a push rod, to cause the bar to deploy distal of the vessel. First and second ends of the filament then may be retracted proximally from outside of a patient's body to retract the bar against the distal lateral surface of the vessel and apply an internal compressive force thereto. The internal compressive force causes coagulation within the punctures that facilitates sealing of the proximal and distal punctures.

After the punctures are effectively sealed, the first end of the filament may be retracted to cause the second end to be pulled through the bore of the bar and from within the patient's body. The bar, which preferably is biodegradable, remains in the patient's body after the filament is removed.

To expedite sealing of the puncture, the first and second ends of the filament may be tensioned within a tensioning device disposed on an exterior surface of a patient's skin. The use of the tensioning device advantageously permits a physician to retract the first and second ends of the filament to apply the internal compressive force via the bar, then lock the filament within the tensioning device so that the physician need not manually retain the compressive force upon the vessel. Additionally, the tensioning device serves to apply an external compressive force to the skin to enhance compression of the vessel.

### Brief Description Of The Drawings

Further features of the invention, its nature and various advantages will be more apparent from the accompanying drawings and the following detailed description of the preferred embodiments, in which:

FIGS. 1A-1D are, respectively, a side view of a first puncture sealing device, side views of alternative puncture sealing devices, and a perspective view of an alternative puncture sealing device provided in accordance with a first embodiment of the present invention;

FIGS. 2A-2B are, respectively, a side view and a side sectional view of apparatus for delivering the puncture sealing device of FIG. 1A;

FIGS. 3A-3F are side sectional views illustrating an exemplary method of using the apparatus of FIGS. 2;

### Detailed Description Of The Invention

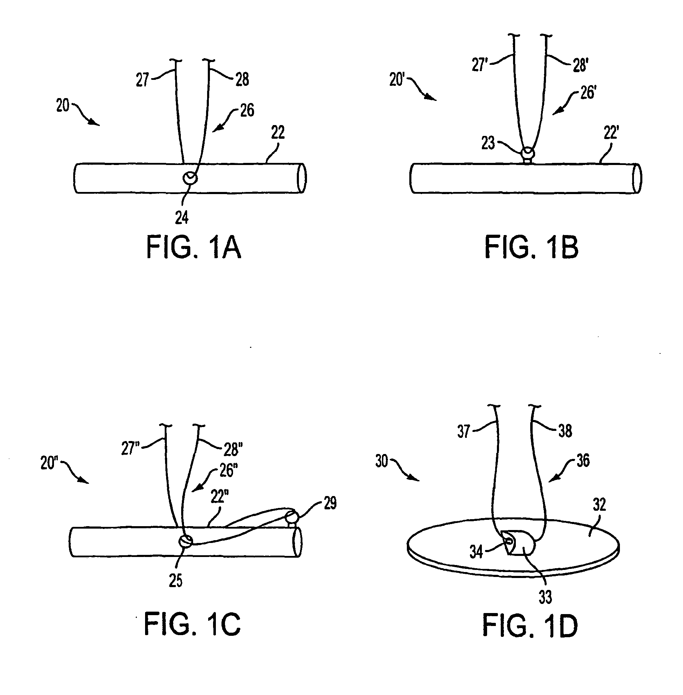

Referring now to FIG. 1A, a first embodiment of a puncture sealing device constructed in accordance with principles of the present invention is described. In FIG. 1A, puncture sealing device 20 comprises cylindrically-shaped bar 22 having proximal and distal ends and bore 24 extending laterally through a central region of bar 22. Bar 22 alternatively may comprise a rectangular or any other cross-section. Filament 26, having first end 27 and second end 28, extends through bore 24 and may be used to manipulate the positioning of bar 22, as described hereinbelow. Preferably, bar 22 comprises a biocompatible plastic or metal alloy, or a biodegradable material such as polyglycolic acid. Filament 26 may comprise a biocompatible wire, or more preferably a conventional suture material, for example, a biodegradable suture material.

In FIG. 1B, alternative puncture sealing device 20' preferably is provided in accordance with bar 22 of FIG. 1A with the exception that bar 22' preferably is solid and comprises first eyelet 23 coupled to a central region of bar 22', e.g., using a solder or weld. Filament 26' extends through first eyelet 23 and may be used to manipulate the positioning of bar 22', as described hereinbelow.

Referring to FIG. 1C, alternative puncture sealing device 20" preferably is provided in accordance with bar 22 of FIG. 1A with the exception that bar 22" comprises central bore 25 extending laterally through a central region of bar 22" and further comprises end eyelet 29 coupled to the distal end of bar 22". Filament 26" preferably extends through central bore 25, then through end eyelet 29, and optionally also may extend back through central bore 25 after passing through eyelet 29, as shown in FIG. 1C. Alternatively, filament 26" may just extend once through central bore 25 and then through end eyelet 29. By retracting either first end 27" or second end 28" of filament 26", it is possible to facilitate horizontal positioning of bar 22" within a patient's tissue for purposes described hereinbelow. As will be apparent to those of skill in the art, bar 22" alternatively may be provided with a central eyelet and an end bore, with central and end eyelets, or with central and end bores.

In FIG. 1D, alternative puncture sealing device 30 is illustrated as comprising substantially flat member 32 having an oval-shaped configuration. Member 32 comprises bore 34 extending through protruding region 33, which preferably is provided in a central region of member 32, as shown in FIG. 1B. Filament 36 having first end 37 and second end 38 extends through bore 34 to perform the functions described hereinbelow. It will be apparent to those skilled in the art that while bars 22 and member 32 of FIGS. 1A-1D are illustratively shown having cylindrical and oval-shaped configurations, respectively, other configurations advantageously may be provided to perform the functions described hereinbelow.

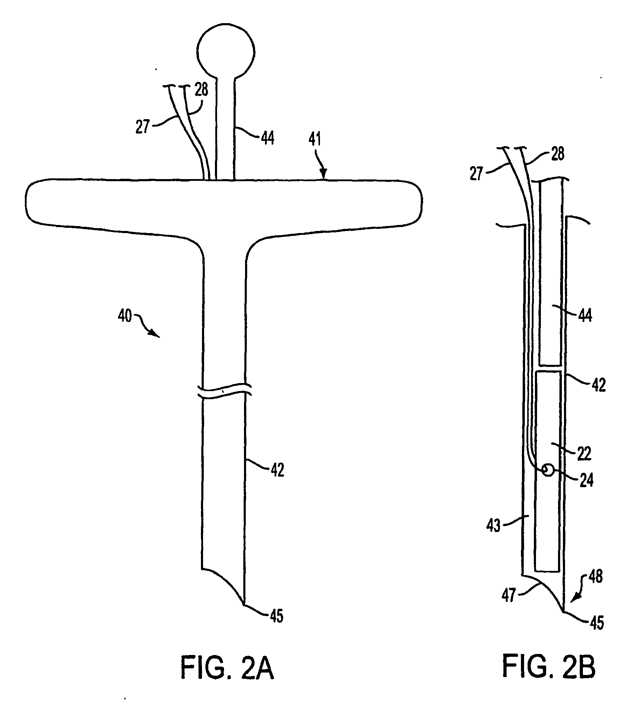

Referring now to FIGS. 2, puncture sealing apparatus 40 of the first embodiment of the present invention preferably comprises delivery sheath 42 having proximal and distal ends and lumen 43 extending therebetween. The proximal end of delivery sheath 42 preferably comprises optional handle 41 that is configured to be grasped by a physician. Distal end 48 of delivery sheath 42 comprises sharpened tip 45 and opening 47, which is in fluid communication with lumen 43, as shown in FIG. 2B. Distal end 48 may comprise, for example, a standard hypodermic needle attached to handle 41. Distal end 48 preferably comprises a significantly smaller cross sectional area than optional handle 41 of delivery sheath 42. Bar 22 and filament 26 of FIG. 1A preferably are used in conjunction with delivery sheath 42, as described hereinbelow.

Apparatus 40 preferably further comprises push rod 44 having proximal and distal ends. Delivery sheath 42 is sized so that bar 22, push rod 44, and first and second ends 27 and 28 of filament 26 may be provided in a contracted state within lumen 43. In the contracted state, the distal end of push rod 44 is disposed just proximal of bar 22.

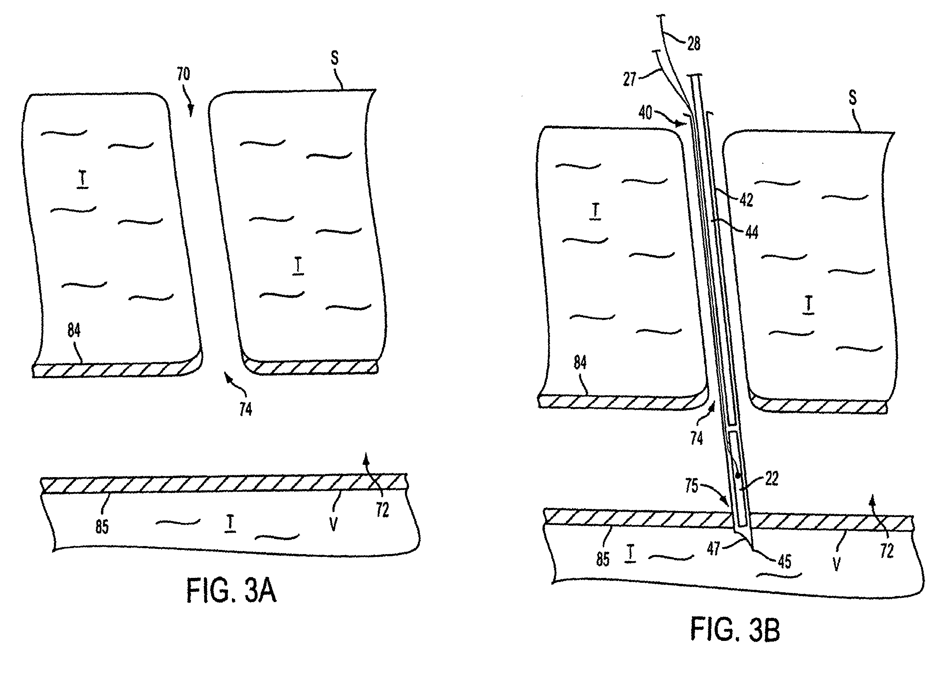

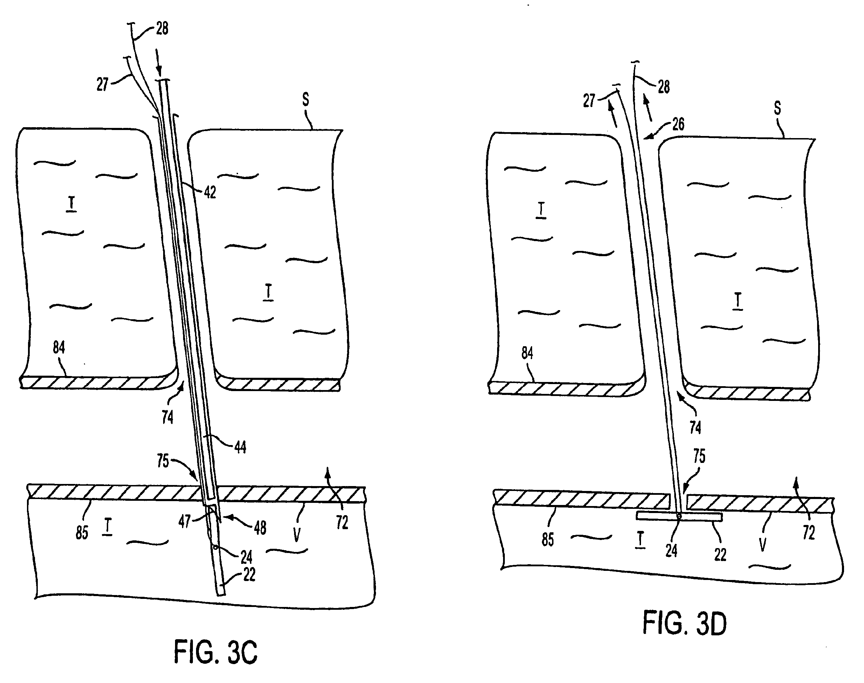

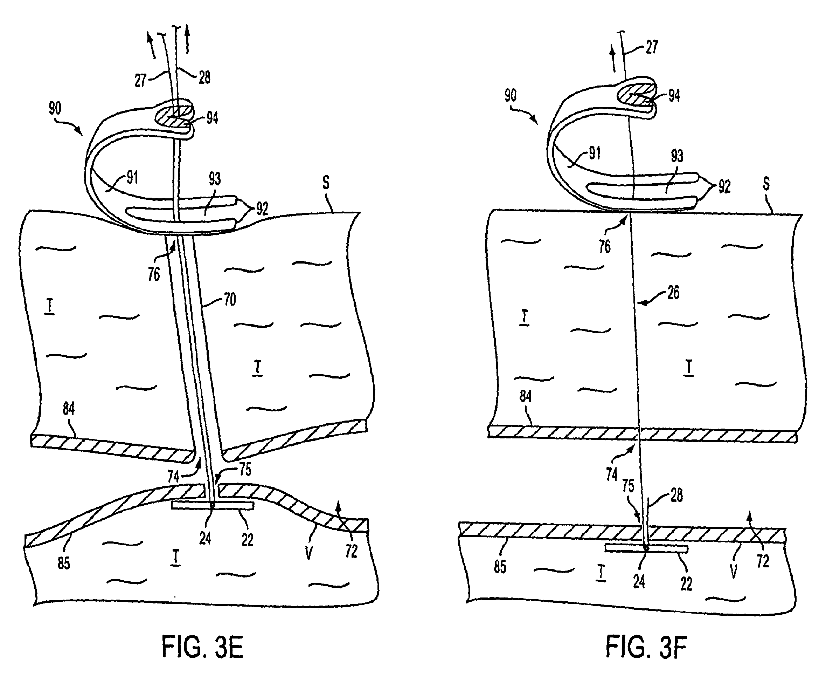

Referring now to FIGS. 3, an exemplary method of using puncture sealing apparatus 40 of FIGS. 2 to seal a puncture in a vessel is described. In FIG. 3A, proximal puncture 74 has been formed in proximal lateral surface 84 of vessel **V**, which is situated within tissue **T.** Puncture tract 70 has been formed and permits fluid communication between an exterior surface of a patient's body and lumen 72 of vessel **V.** Puncture tract 70 may have been formed, for example, as a means for introducing a guidewire and/or catheter into vessel **V** to perform a variety of medical procedures.

Referring now to FIG. 3B, a first step for using puncture sealing apparatus 40 of FIGS. 2 in accordance with principles of the present invention is described. Sharpened tip 45 at the distal end of delivery sheath 42 is inserted into a patient's body, preferably via preexisting puncture tract 70 of FIG. 3A. Specifically, sharpened tip 45 is inserted through skin **S,** tissue **T,** proximal puncture 74, and through lumen 72 of vessel **V.** To ensure that sharpened tip 45 of apparatus 40 does not accidentally pierce tissue T during delivery, apparatus 40 optionally may be delivered through an external sheath, such as an introducer or guiding catheter.

Sharpened tip 45 further is advanced distally through lumen 72 to pierce through a distal wall of vessel **V,** such that sharpened tip 45 is again disposed within tissue **T** distal of vessel **V,** as shown in FIG. 3B. The piercing of the distal wall of vessel **V** forms distal puncture 75 in distal lateral surface 85 of vessel **V,** which is substantially diametrically opposing proximal puncture 74 in proximal lateral surface 84. Distal puncture 75 preferably is significantly smaller than proximal puncture 74, for example, twice as small to an order of magnitude or more smaller in cross-sectional diameter.

Referring to FIG. 3C, with distal end 48 of delivery sheath 42 positioned at a desired distance distal of distal puncture 75, the proximal end of push rod 44 is advanced distally by a physician to cause the distal end of push rod 44 to abut the proximal end of bar 22. Push rod 44 is advanced distally until bar 22 is disposed distal of opening 47. Delivery sheath 42 and push rod 44 then may be retracted proximally and removed from the patient's body.

After bar 22 is ejected from delivery sheath 42, filament 26 may be pulled slightly proximally to cause bar 22 to assume an orientation that is substantially parallel to a longitudinal axis of vessel **V,** as shown in FIG. 3D. When bar 22" of FIG. 1C is used in place of bar 22, central bore 25 and end eyelet 29 may be utilized to assist in urging bar 22" to an orientation that is substantially parallel to the vessel when either or both ends of filament 26" are retracted. In FIG. 3D, first and second ends 27 and 28 of filament 26 are retracted further proximally to retract bar 22 toward distal lateral surface 85 of vessel **V.**

Referring now to FIG. 3E, tensioning device 90 preferably is used in conjunction with apparatus 40 of the present invention. Tensioning device 90 is similar in structure to a tensioning device previously commercially marketed under the trade name "BioDISC" by BioInterventional Corp. of Pleasanton, CA. Tensioning device 90 comprises upright 91 having legs 92 and grip 94. Grip 94 may comprise a V-shaped groove in an elastomeric block and retains tension on filament 26 when ends 27 and 28 are pulled distally.

Legs 92 of tensioning device 90 are placed atop an exterior surface of skin S so that space 93, formed between legs 92, is positioned substantially over skin puncture 76. First and second ends 27 and 28 of filament 26 are engaged in grip 94, and may be retracted proximally while legs 92 maintain contact with skin **S**, as shown in FIG. 3E. The retraction of filament 26 causes bar 22 to provide an internal compressive force upon distal lateral surface 85 of vessel **V,** while legs 92 provide an external compressive force upon proximal lateral surface 84 through tissue **T** during tensioning of filament 26.

When the desired tension is provided in filament 26, grip 94 retains ends 27 and 28 in a tensioned state. Advantageously, this permits the internal and external compressive forces described hereinabove to be applied to vessel **V** without requiring a physician to manually hold ends 27 and 28 of filament 26 for an extended period of time.

The compressive forces imposed upon vessel **V** cause lumen 72 to narrow locally, which in turn causes coagulation of blood in the vicinity of punctures 74 and 75. Over a period of time, the coagulation occurring within punctures 74 and 75 results in a reduction in diameter of the punctures and halts blood loss from the vessel. Advantageously, the use of bar 22 in conjunction with tensioning device 90 allows compressive forces to be applied to vessel **V** from substantially diametrically opposing surfaces of vessel **V** to facilitate closure of punctures 74 and 75. If desired, filament ends 27 and 28 may be intermittently disengaged from grip 94 throughout the procedure to relieve the tensile force imposed upon filament 26 to temporarily reduce the compressive forces applied to vessel **V.**

If desired, collagen or a biocompatible gel or polymer, such as a water swellable gel or a biodegradable polymer like Polyethylene Glycol ("PEG"), may be injected into puncture tract 70 to reduce a diameter of the puncture tract either before, during or after the time in which the internal compressive force is applied to distal lateral surface 85. If the use of collagen, gels or polymers is employed, it is preferred that the agent is injected into puncture tract 70 while filament 26 is tensioned within tensioning device 90.

Referring now to FIG. 3F, the compressive forces applied to vessel **V** have caused a significant reduction in the diameter of punctures 74 and 75 to effectively seal the punctures. Filament 26 then may be released from grip 94 to relieve the tensile forces imposed upon filament 26. A physician may proximally retract first end 27 of filament 26 to cause second end 28 to be pulled in a distal direction through skin puncture 76, proximal and distal punctures 74 and 75, and through bore 24 of bar 22. First end 27 further is retracted proximally to remove second end 28 from the patient's body, thereby leaving bar 22 disposed within tissue **T.** If bar 22 is manufactured from a biocompatible material such as polyglycolic acid, it will be resorbed by the patient's body.

In an alternative approach for sealing puncture 74 of FIGS. 3, sharpened tip 45 of sheath 42 may initially pierce substantially deeper into tissue **T,** i.e., to a location substantially distal of distal lateral surface 85 of vessel **V.** Accordingly, when bar 22 is ejected from sheath 42, bar 22 is disposed at a location within tissue **T** that is not in close proximity to distal lateral surface 85. Using this approach, when filament 26 is placed in tension, tissue **T,** as opposed to bar 22 directly, applies the internal compressive force upon distal lateral surface 85. This approach advantageously may reduce trauma to distal lateral surface 85 of vessel **V** when compressive forces are imposed as described hereinabove.

Moreover, it will be apparent to those skilled in the art that the figures accompanying the preferred embodiments are provided only for the sake of illustration and are not drawn to scale. For example, it is expected that the diameter of distal end 48 of delivery sheath 42 of FIGS. 3B-3C, as well as the diameters of all apparatus disposed therein, will be significantly smaller than the diameter of puncture **P** or vessel **V.** Additionally, in FIGS. 3, the diameter of distal puncture 75 is expected to be significantly smaller than the diameter of proximal puncture 74.

While preferred illustrative embodiments of the invention are described above, it will be apparent to one skilled in the art that various changes and modifications may be made therein without departing from the invention. The appended claims are intended to cover all such changes and modifications that fall within the scope of the invention.

## Claims

1. Apparatus for facilitating sealing of a puncture (74) formed in a proximal lateral surface (84) of a vessel (V) in a patient's body, the apparatus comprising:
a bar (22, 22") having proximal and distal ends and a first bore (24, 25) extending laterally there-through; and
a filament (26, 26") having a first end (27, 27") and a second end (28, 28"), the filament (26, 26") disposed through the first bore (24, 25) such that the first and second ends (27, 27"; 28, 28") extend in the same direction with respect to said bore (24, 25)
wherein the bar (22, 22") is configured to apply a compressive force upon a distal lateral surface (85) of a vessel (V),
**characterized in that** the filament (26, 26") is adapted to run freely through the first bore (24, 25), and **in that** the first proximal end (27, 27") of the filament (26, 26") is adapted to be retracted to cause the second end (28, 28") of the filament to be pulled through the first bore (24, 25) of the bar (22, 22") and from within the patient's body.

2. The apparatus of claim 1 further comprising a delivery sheath (42) having proximal and distal ends, a lumen (43) extending there-between and a sharpened tip (45) at the distal end, wherein the lumen (43) is configured to contain the bar (22, 22") and filament (26, 26").

3. The apparatus of claim 2 further comprising a push rod (44) disposed in the lumen (43) proximal of the bar (22, 22").

4. The apparatus of claim 1, 2 or 3 wherein the bar (22, 22") has a shape chosen from the group consisting of cylindrical shapes, rectangular shapes, and oval shapes.

5. The apparatus of claim 1, 2, 3 or 4 wherein the bar (22, 22") comprises a biodegradable material.

6. The apparatus of claim 1, 2, 3, 4 or 5 further comprising a tensioning device (90) configured to hold the filament (26, 26") in a tensioned state.

7. The apparatus of claim 6 wherein the tensioning device (90) comprises:
an upright (91) having upper and lower ends;
a plurality of legs (92) attached to the lower end; and
a grip (94) affixed to the upper end.

8. The apparatus of claim 7 wherein the grip (94) comprises a V-shaped groove formed in an elastomeric material.

9. The apparatus of any one of claims 1 to 8 wherein the first bore (24, 25) is disposed in a central region of the bar (22, 22").

10. The apparatus of any one of claims 1 to 9 further comprising an eyelet (29) coupled to the bar (22"), wherein the filament (26") is disposed through both the first bore and the eyelet (23).

11. The apparatus of claim 10 wherein the first bore (25) is disposed in a central region of the bar (22"), and the eyelet (29) is coupled to a distal region of the bar.

12. The apparatus of claim 10 or 11 further comprising an eyelet (23) coupled to the bar, wherein the eyelet (23) is coupled to a central region of the bar, and the first bore (24, 25) is disposed in a distal region of the bar.

13. The apparatus of any one of claims 1 to 12 further comprising a second bore extending laterally through the bar, wherein the filament is disposed through both the first bore and the second bore.

14. The apparatus of claim 13 wherein the first bore (24, 25) is disposed in a central region of the bar (22, 22"), and the second bore is disposed in a distal region of the bar.

15. Apparatus for facilitating sealing of a puncture (74) formed in a proximal lateral surface (84) of a vessel (V) in a patient's body, the apparatus comprising:
a bar (22', 32) having proximal and distal ends and a first eyelet (23, 33) coupled to the bar (22', 32); and
a filament (26', 36) having a first end (27', 37) and a second end (28', 38), the filament (26', 36) disposed through the first eyelet (23, 33) such that the first and second ends (27', 37; 28', 38) extend in the same direction with respect to said eyelet (23, 33)
wherein the bar (22', 32) is configured to apply a compressive force upon a distal lateral surface (85) of a vessel,
**characterized in that** the filament (26', 36) is adapted to run freely through the first eyelet (23, 33), and **in that** the first proximal end (27', 37) of the filament (26', 36) is adapted to be retracted to cause the second end (28', 38) of the filament to be pulled through the first eyelet (23, 33) of the bar (22', 32) and from within the patient's body.

16. The apparatus of claim 15 wherein the first eyelet (23, 33) is coupled to a central region of the bar (22', 32).

17. The apparatus of claim 15 or 16 wherein the bar further comprises a second eyelet coupled to the bar (22', 32), wherein the filament is disposed through the first eyelet (23, 33) and the second eyelet.

18. The apparatus of claim 17 wherein the first eyelet (23, 33) is coupled to a central region of the bar (22', 32), and the second eyelet is coupled to a distal region of the bar.

19. The apparatus of claim 15,16, 17 or 18 further comprising a delivery sheath (42) having proximal and distal ends, a lumen (43) extending there-between and a sharpened tip (45) at the distal end, wherein the lumen (43) is configured to contain the bar (22', 32) and filament (26', 36).

20. The apparatus of claim 19 further comprising a push rod (44) disposed in the lumen (43) of the delivery sheath (42) proximal of the bar (22', 32).

21. The apparatus of any one of claims 15 to 20 wherein the bar (22', 32) comprises a biodegradable material.

22. The apparatus of any one of claims 15 to 21 further comprising a tensioning device (90) configured to hold the filament (26', 36) in a tensioned state.

## Patentansprüche

1. Gerät (50) zur Erleichterung des Verschließens einer Punktion (74), die in einer proximalen lateralen Oberfläche (84) eines Gefäßes (V) im Körper eines Patienten ausgebildet ist, wobei das Gerät umfasst:
einen Stab (22, 22"), welcher proximale und distale Enden und eine erste Bohrung (24, 25), die sich lateral durch den Stab hindurch erstreckt, aufweist; und
ein Filament (26, 26"), welches ein erstes Ende (27, 27") und ein zweites Ende (28, 28") aufweist, wobei das Filament (26, 26") durch die erste Bohrung (24, 25) derart geführt ist, dass sich die ersten und zweiten Enden (27, 27"; 28, 28") in Bezug auf die Bohrung (24, 25) in die gleiche Richtung erstrecken,
wobei der Stab (22, 22") zum Ausüben einer Druckkraft auf eine distale laterale Oberfläche (85) eines Gefäßes (V) konfiguriert ist,
**dadurch gekennzeichnet, dass** das Filament (26, 26") zum freien Laufen durch die erste Bohrung (24, 25) adaptiert ist, und dass das erste proximale Ende (27, 27") des Filaments (26, 26") zum Zurückziehen adaptiert ist, um das Ziehen des zweiten Endes (28, 28") des Filaments durch die erste Bohrung (24, 25) des Stabs (22, 22") hindurch und aus dem Körper des Patienten heraus zu bewirken.

2. Gerät nach Anspruch 1, welches außerdem eine Zuführungshülse (42) umfasst, welche proximale und distale Enden, ein Lumen (43), das sich zwischen den Enden erstreckt, und eine zugespitzte Spitze (45) an dem distalen Ende aufweist, wobei das Lumen zur Aufnahme des Stabs (22, 22") und des Filaments (26, 26") konfiguriert ist.

3. Gerät nach Anspruch 2, welches ferner eine Stößelstange (44) umfasst, die in dem Lumen (43) proximal des Stabs (22, 22") angeordnet ist.

4. Gerät nach Anspruch 1, 2 oder 3, wobei der Stab (22, 22") eine Form aufweist, die ausgewählt ist aus der Gruppe bestehend aus zylindrischen Formen, rechteckigen Formen und ovalen Formen.

5. Gerät nach Anspruch 1, 2, 3 oder 4, wobei der Stab (22, 22") ein biologisch abbaubares Material umfasst.

6. Gerät nach Anspruch 1, 2, 3, 4 oder 5, welches außerdem eine Spannvorrichtung (90) umfasst, die zum Halten des Filaments (26, 26") in einem gespannten Zustand konfiguriert ist.

7. Gerät nach Anspruch 6, wobei die Spannvorrichtung (90) umfasst:
eine Stütze (91), welche ein oberes und ein unteres Ende aufweist;
eine Anzahl von Beinen (92), die mit dem unteren Ende verbunden sind; und
einen Griff (94) der an dem oberen Ende befestigt ist.

8. Gerät nach Anspruch 7, wobei der Griff (94) eine V-förmige Aussparung umfasst, die in einem elastomeren Material ausgebildet ist.

9. Gerät nach irgendeinem der Ansprüche 1 bis 8, wobei die erste Bohrung (24, 25) in einem zentralen Bereich des Stabs (22, 22") angeordnet ist.

10. Gerät nach irgendeinem der Ansprüche 1 bis 9, welches außerdem eine Öse (29) umfasst, die mit dem Stab (22") verbunden ist, wobei das Filament (26") sowohl durch die erste Bohrung als auch die Öse (23) geführt ist.

11. Gerät nach Anspruch 10, wobei die erste Bohrung (25) in einem zentralen Bereich des Stabs (22") angeordnet ist, und die Öse (29) mit einem distalen Bereich des Stabs verbunden ist.

12. Gerät nach Anspruch 10 oder 11, welches außerdem eine Öse (23) umfasst, die mit dem Stab verbunden ist, wobei die Öse (23) mit einem zentralen Bereich des Stabs verbunden ist, und die erste Bohrung (24, 25) in einem distalen Bereich des Stabs angeordnet ist.

13. Gerät nach irgendeinem der Ansprüche 1 bis 12, welches ferner eine zweite Bohrung umfasst, die sich lateral durch den Stab hindurch erstreckt, wobei das Filament sowohl durch die erste Bohrung als auch durch die zweite Bohrung geführt ist.

14. Gerät nach Anspruch 13, wobei die erste Bohrung (24, 25) in einem zentralen Bereich des Stabs (22, 22") angeordnet ist, und die zweite Bohrung in einem distalen Bereich des Stabs angeordnet ist.

15. Gerät (50) zur Erleichterung des Verschließens einer Punktion (74), die in einer proximalen lateralen Oberfläche (84) eines Gefäßes (V) im Körper eines Patienten ausgebildet ist, wobei das Gerät umfasst:
einen Stab (22', 32), welcher proximale und distale Enden und eine erste Öse (23, 33), die mit dem Stab (22', 32) verbunden ist, aufweist; und
ein Filament (26', 36), welches ein erstes Ende (27', 37) und ein zweites Ende (28', 38) aufweist, wobei das Filament (26', 36) durch die erste Öse (23, 33) derart geführt ist, dass sich die ersten und zweiten Enden (27', 37; 28', 38) in Bezug auf die Bohrung (23, 33) in die gleiche Richtung erstrecken,
wobei der Stab (22', 32) zum Ausüben einer Druckkraft auf eine distale laterale Oberfläche (85) eines Gefäßes (V) konfiguriert ist,
**dadurch gekennzeichnet, dass** das Filament (26',36) zum freien Laufen durch die erste Öse (23, 33) adaptiert ist, und dass das erste proximale Ende (27', 37) des Filaments (26', 36) zum Zurückziehen adaptiert ist, um das Ziehen des zweiten Endes (28', 38) des Filaments durch die erste Öse (23, 33) des Stabs (22', 32) hindurch und aus dem Körper des Patienten heraus zu bewirken.

16. Gerät nach Anspruch 15, wobei die erste Öse (23, 33) mit einem zentralen Bereich des Stabs (22', 32) verbunden ist.

17. Gerät nach Anspruch 15 oder 16, wobei der Stab ferner eine zweite Öse umfasst, die mit dem Stab (22', 32) verbunden ist, wobei das Filament durch die erste Öse (23, 33) und die zweite Öse geführt ist.

18. Gerät nach Anspruch 17, wobei die erste Öse (23, 33) mit einem zentralen Bereich des Stabs (22', 32) verbunden ist, und die zweite Öse mit einem distalen Bereich des Stabs verbunden ist.

19. Gerät nach Anspruch 15, 16, 17 oder 18, welches außerdem eine Zuführungshülse (42) umfasst, welche proximale und distale Enden, ein Lumen (43), das sich zwischen den Enden erstreckt und eine zugespitzte Spitze (45) an dem distalen Ende aufweist, wobei das Lumen (43) zur Aufnahme des Stabs (22', 32) und des Filaments (26', 36) konfiguriert ist.

20. Gerät nach Anspruch 19, welches ferner eine Stößelstange (44) umfasst, die in dem Lumen (43) der Zuführungshülse (42) proximal des Stabs (22', 32) angeordnet ist.

21. Gerät nach irgendeinem der Ansprüche 15 bis 20, wobei der Stab (22', 32) ein biologisch abbaubares Material umfasst.

22. Gerät nach irgendeinem der Ansprüche 15 bis 21, welches außerdem eine Spannvorrichtung (90) umfasst, die zum Halten des Filaments (26', 36) in einem gespannten Zustand konfiguriert ist.

## Revendications

1. Appareil destiné à faciliter la fermeture d'une ponction (74) formée dans une surface latérale proximale (84) d'un vaisseau (V) dans le corps d'un patient, l'appareil comprenant :
une barre (22, 22") ayant des extrémités proximale et distale et un premier alésage (24, 25) s'étendant latéralement à travers celle-ci ; et
un filament (26, 26") ayant une première extrémité (27, 27") et une deuxième extrémité (28, 28"), le filament (26, 26") disposé dans l'alésage (24, 25) de telle manière que les première et deuxième extrémités (27, 27" ; 28, 28") s'étendent dans la même direction relativement au dit alésage (24, 25)
la barre (22, 22") étant configurée pour appliquer une force de compression sur une surface latérale distale (85) d'un vaisseau (V),
**caractérisé en ce que** le filament (26, 26") est adapté pour se déplacer librement dans le premier alésage (24, 25) et **en ce que** la première extrémité proximale (27, 27") du filament (26, 26") est adaptée pour être rétractée et entraîner la traction de la deuxième extrémité (28, 28") du filament dans le premier alésage (24, 25) de la barre (22, 22") et de l'intérieur du corps du patient.

2. Appareil selon la revendication 1, qui comprend en outre une gaine de délivrance (42) ayant des extrémités proximale et distale, une lumière (43) s'étendant entre celles-ci et une extrémité affûtée (45) au niveau de l'extrémité distale, la lumière (43) étant configurée pour contenir la barre (22, 22") et le filament (26, 26").

3. Appareil selon la revendication 2, qui comprend en outre une tige poussoir (44) disposée dans la lumière (43) proximale de la barre (22, 22").

4. Appareil selon la revendication 1, 2 ou 3 dans lequel la barre (22, 22") a une forme choisie dans le groupe constitué par les formes cylindriques, les formes rectangulaires, et les formes ovales.

5. Appareil selon la revendication 1, 2, 3 ou 4, dans lequel la barre (22, 22") comprend un matériau biodégradable.

6. Appareil selon la revendication 1, 2, 3, 4 ou 5, qui comprend en outre un dispositif de mise en tension (90) configuré pour maintenir le filament (26, 26") dans un état de tension.

7. Appareil selon la revendication 6, dans lequel le dispositif de mise en tension (90) comprend :
un montant (91) ayant des extrémités supérieure et inférieure ;
une pluralité de pattes (92) fixées à l'extrémité inférieure ; et
un point de préhension (94) fixé à l'extrémité supérieure.

8. Appareil selon la revendication 7, dans lequel le point de préhension (94) comprend un sillon en forme de V formé dans un matériau élastomère.

9. Appareil selon l'une quelconque des revendications 1 à 8, dans lequel le premier alésage (24, 25) est disposé dans une région centrale de la barre (22, 22").

10. Appareil selon l'une quelconque des revendications 1 à 9, qui comprend en outre un oeillet (29) couplé à la barre (22"), le filament (26") étant disposé à la fois dans le premier alésage et dans l'oeillet (23).

11. Appareil selon la revendication 10, dans lequel le premier alésage (25) est disposé dans une région centrale de la barre (22"), et l'oeillet (29) est couplé à une région distale de la barre.

12. Appareil selon la revendication 10 ou 11, qui comprend en outre un oeillet (23) couplé à la barre, l'oeillet (23) étant couplé à une région centrale de la barre et le premier alésage (24, 25) étant disposé dans une région distale de la barre.

13. Appareil selon l'une quelconque des revendications 1 à 12, qui comprend en outre un deuxième alésage s'étendant latéralement dans la barre, le filament étant disposé à la fois dans le premier alésage et dans le deuxième alésage.

14. Appareil selon la revendication 13, dans lequel le premier alésage (24, 25) est disposé dans une région centrale de la barre (22, 22"), et le deuxième alésage est disposé dans une région distale de la barre.

15. Appareil destiné à faciliter la fermeture d'une ponction (74) formée dans une surface latérale proximale (84) d'un vaisseau (V) dans le corps d'un patient, l'appareil comprenant :
une barre (22', 32) ayant des extrémités proximale et distale et un premier oeillet (23, 33) couplé à la barre (22', 32) ; et
un filament (26', 36) ayant une première extrémité (27', 37) et une deuxième extrémité (28', 38), le filament (26', 36) disposé dans le premier oeillet (23, 33) de telle manière que les première et deuxième extrémités (27', 37 ; 28', 38) s'étendent dans la même direction relativement au dit oeillet (23, 33) ;
la barre (22', 32) étant configurée pour appliquer une force de compression sur une surface latérale distale (85) d'un vaisseau ;
**caractérisé en ce que** le filament (26', 36) est adapté pour se déplacer librement dans le premier oeillet (23, 33) et **en ce que** la première extrémité proximale (27', 37) du filament (26', 36) est adaptée pour être rétractée et entraîner la traction de la deuxième extrémité (28', 38) du filament dans le premier oeillet (23, 33) de la barre (22', 32) et de l'intérieur du corps du patient.

16. Appareil selon la revendication 15, dans lequel le premier oeillet (23, 33) est couplée à une région centrale de la barre (22', 32).

17. Appareil selon la revendication 15 ou 16, dans lequel la barre comprend en outre un deuxième oeillet couplé à la barre (22', 32), le filament étant disposé dans le premier oeillet (23, 33) et dans le deuxième oeillet.

18. Appareil selon la revendication 17, dans lequel le premier oeillet (23, 33) est couplé à une région centrale de la barre (22', 32), et le deuxième oeillet est couplé à une région distale de la barre.

19. Appareil selon la revendication 15, 16, 17 ou 18 qui comprend en outre une gaine de délivrance (42) ayant des extrémités proximale et distale, une lumière (43) s'étendant entre celles-ci et une extrémité affûtée (45) au niveau de l'extrémité distale, la lumière (43) étant configurée pour contenir la barre (22', 32) et le filament (26', 36).

20. Appareil selon la revendication 19, qui comprend en outre une tige poussoir (44) disposée dans la lumière (43) de la gaine de délivrance (42) proximale de la barre (22', 32).

21. Appareil selon l'une quelconque des revendications 15 à 20, dans lequel la barre (22', 32) comprend un matériau biodégradable.

22. Appareil selon l'une quelconque des revendications 15 à 21, comprenant en outre un dispositif de mise en tension (90) configuré pour maintenir le filament (26', 36) dans un état de tension.
